# EUROPÄISCHE PATENTSCHRIFT

(11) **EP 1 449 511 B1**
(45) Veröffentlichungstag und Bekanntmachung des Hinweises auf die Patenterteilung: **04.10.2006**
(21) Anmeldenummer: 04010332.7
(22) Anmeldetag: 19.05.2000
(51) Int. Cl.: A61K 8/35, A61Q 19/00

(54) **Verwendung von Idebenon zum Schutz von Zubereitungen**
Use of Idebenone for the preservation of compositions
Utilisation d'Idebenone pour la conservation des compositions

(30) Priorität: 09.07.1999 DE 19932197
(43) Veröffentlichungstag der Anmeldung: 25.08.2004
(62) Teilanmeldung aus: 00945551.0
(73) Patentinhaber: Neudecker, Birgit, 34233 Fuldatal-Rothwesten (DE); Wieland, Eberhard, 37077 Göttingen (DE); Diedrich, Falko, 34233 Fuldatal-Rothwesten (DE)
(72) Erfinder: Neudecker, Birgit, 34233 Fuldatal-Rothwesten (DE); Wieland, Eberhard, 37077 Göttingen (DE); Diedrich, Falko, 34233 Fuldatal-Rothwesten (DE)
(74) Vertreter: TER MEER - STEINMEISTER & PARTNER GbR

(56) Entgegenhaltungen:
- WO-A-94/20068
- DATABASE CAPLUS [Online] CHEMICAL ABSTRACTS SERVICE, COLUMBUS, OHIO, US; WIELAND, EBERHARD ET AL: "Idebenone protects hepatic microsomes against oxygen radical-mediated damage in organ preservation solutions" XP002283957 gefunden im STN Database accession no. 1995:881853 & TRANSPLANTATION , 60(5), 444-51 CODEN: TRPLAU; ISSN: 0041-1337, 1995,
- PATENT ABSTRACTS OF JAPAN Bd. 014, Nr. 047 (C-0682), 29. Januar 1990 (1990-01-29) & JP 01 279818 A (TAKEDA CHEM IND LTD), 10. November 1989 (1989-11-10)
- PATENT ABSTRACTS OF JAPAN Bd. 1999, Nr. 09, 30. Juli 1999 (1999-07-30) & JP 11 116470 A (TAKEDA CHEM IND LTD), 27. April 1999 (1999-04-27)
- DATABASE WPI Week 199219 Derwent Publications Ltd., London, GB; AN 1992-157308 XP002148788 "External adhesive drug - contg. base agent of fatty acids, animal and plant fats and oils, polyhydric alcohol, and analgesics, antial drugs, etc." & JP 04 099719 A (RIDO CHEMICAL) 31. März 1992 (1992-03-31)

## Beschreibung

Die vorliegende Erfindung betrifft die Verwendung von Idebenon und/oder seinen Derivaten in kosmetischen und topischen dermatologische Zubereitungen zum Schutze kosmetischer Zubereitungen selbst. bzw. zum Schutz der Bestandteile kosmetischer Zubereitungen vor schädlichen Oxidationsprozessen. Solche kosmetischen und dermatologischen Zubereitungen mit einem wirksamen Gehalt an Idebenon und/oder seinen Derivaten fördern über eine Unterstützung der Zellatmung oder Stabilisierung der Mitochondrienmembranen die Regeneration und Vitalität von Hautzellen.

Die vorliegende Erfindung betrifft ferner Antioxidantien, und hier bevorzugt solche, welche in hautpflegenden kosmetischen oder dermatologischen Zubereitungen eingesetzt werden. Insbesondere betrifft die Erfindung auch kosmetische und dermatologische Zubereitungen, solche Antioxidantien in Kombination mit Idebenon und/oder seine Derivate enthaltend. Weiterhin betrifft die vorliegende Erfindung kosmetische und dermatologische Zubereitungen zur Prophylaxe und Behandlung kosmetischer und dermatologischer Hautveränderungen wie z. B. der Hautalterung, und hier insbesondere der durch oxidative oder degenerative Prozesse hervorgerufenen Hautalterung.

Ferner betrifft die vorliegende Erfindung auch kosmetische und topische dermatologische Zubereitungen, in denen Idebenon und seine Derivate zusammen mit Glykosaminoglykanen und ihren Salzen, insbesondere Hyaluronsäure mit einem Molekülgewicht von 1 bis 1000000 und ihren Salzen, oder Hyaluronidase-Inhibitoren wie z.B. Inter-alpha-Trypsin-Inhibitor eingesetzt werden. Zusätzlich betrifft die vorliegende Erfindung auch kosmetische und dermatologische Zubereitungen, in denen Idebenon und/oder seine Derivate mit Glykosaminoglykanen und/oder ihren Salzen, insbesondere Hyaluronsäure mit einem Molekülgewicht von 1 bis 1000000 und ihren Salzen oder Hyaluronidase-Inhibitoren wie z. B. Inter-alpha-Trypsin-Inhibitor verestert werden.

Weiterhin betrifft die vorliegende Erfindung Wirkstoffe und Zubereitungen, solche Wirkstoffe enthaltend, zur Prophylaxe erythematöser, entzündlicher, allergischer oder autoimmunreaktiver Erscheinungen, insbesondere Dermatosen.

Die vorliegende Erfindung betrifft ferner Wirkstoffkombinationen und Zubereitungen, die zur Prophylaxe und Behandlung der lichtempfindlichen Haut, insbesondere der Photodermatosen, dienen.

Die schädigende Wirkung des ultravioletten Teils der Sonnenstrahlung auf die Haut ist allgemein bekannt.

Während Strahlen mit einer Wellenlänge, die kleiner als 290 nm ist (der sogenannte UVC-Bereich), von der Ozonschicht in der Erdatmosphäre absorbiert werden, verursachen Strahlen im Bereich zwischen 290 nm und 320 nm, dem sogenannten UVB-Bereich, ein Erythem, einen einfachen Sonnenbrand oder sogar mehr oder weniger starke Verbrennungen.

Als ein Maximum der Erythemwirksamkeit des Sonnenlichtes wird der engere Bereich um 308 nm angegeben. Zum Schutz gegen UVB-Strahlung sind zahlreiche Verbindungen bekannt bei denen es sich um Derivate des 3-Benzylidencamphers, der 4-Aminobenzoesäure, der Zimtsäure, der Salicylzäure, des Benzophenons sowie auch des 2-Phenylbenzimidazols handelt.

Auch für den Bereich zwischen etwa 320 nm und etwa 400 nm, des sogenannten UVA-Bereich, ist es wichtig, Filtersubstanzen zur Verfügung zu haben, da dessen Strahlen Reaktionen bei lichtempfindlicher Haut hervorrufen können. Es ist erwiesen, daß UVA-Strahlung zu einer Schädigung der elastischen und kollagenen Fasern des Bindegewebes führt, was die Haut vorzeitig altern läßt, und daß sie als Ursache zahlreicher phototoxischer und photoallergischer Reaktionen zu sehen ist. Der schädigende Einfluß der UVB-Strahlung kann durch UVA-Strahlung verstärkt werden. Es ist weiterhin erwiesen, daß durch UVA- und UVB-Strahlung ein Verbrauch lipophiler Antioxidantien, z. B. alpha-Tocopherol, in der Haut ausgelöst wird (Thiele et al., J Invest Dermatol 110, S. 756ff (1998)).

Zum Schutz gegen die Strahlen des UVA-Bereichs wurden daher gewisse Derivate des Dibenzoylmethans verwendet, deren Photostabilität (Int. J. Cosm. Science 10, 53 (1988)) nicht in ausreichendem Maße gegeben ist. Die UV-Strahlung kann aber auch zu photochemischen Reaktionen führen, wobei dann die photochemischen Reaktionsprodukte in den Hautmetabolismus eingreifen.

Vorwiegend handelt es sich bei solchen photochemischen Reaktionsprodukten um radikalische Verbindungen, beispielsweise Hydroxyradikale. Auch undefinierte radikalische Photoprodukte, welche in der Haut selbst entstehen, können aufgrund ihrer hohen Reaktivität unkontrollierte Folgereaktionen auslösen. Aber auch Singulettsauerstoff, ein nichtradikalischer angeregter Zustand des Sauerstoffmoleküls kann bei UV-Bestrahlung auftreten, ebenso kurzlebige Epoxide und viele andere. Singulettsauerstoff beispielsweise zeichnet sich gegenüber dem normalerweise vorliegenden Triplettsauerstoff (radikalischer Grundzustand) durch gesteigerte Reaktivität aus. Allerdings existieren auch angeregte, reaktive (radikalische) Triplettzustände des Sauerstoffmoleküls. Weiterhin kommt es zum Auftreten von Lipidperoxidationsprodukten wie z. B. Hydroperoxiden und Aldehyden, wobei erstere ihrerseits radikalische Kettenreaktionen auslösen können und denen insgesamt cytotoxische Eigenschaften zugeschrieben werden müssen (Michiels and Remacle, Toxicology, 66, 225ff (1990)).

Ferner zählt UV-Strahlung zur ionisierenden Strahlung. Es besteht also das Risiko, daß auch ionische Spezies bei UV-Exposition entstehen, welche dann ihrerseits oxidativ in die biochemischen Prozesse einzugreifen vermögen.

Um diesen Reaktionen vorzubeugen, können den kosmetischen bzw. dermatologischen Formulierungen zusätzliche Antioxidantien und/oder Radikalfänger einverleibt werden.

Es ist bereits vorgeschlagen worden, Vitamin E, eine Substanz mit bekannter antioxidativer Wirkung in Lichtschutzformulierungen einzusetzen, dennoch bleibt auch hier die erzielte Wirkung weit hinter der erhofften zurück. Insbesondere ist hierbei die Entstehung prooxidativer Abbauprodukte des Tocopherols zu erwähnen, die bei Einsatz von Idebenon und/oder seiner Derivate nicht auftreten, bzw. bei Kombination von Tocopherol und Idebenon und/oder seinen Derivaten zuverlässig abgefangen werden können.

In eigenen Vorversuchen wurde Idebenon im Makrophagen-LDL-Oxidationssystem als Antioxidans vergleichend zu alpha-Tocopherol, Ascorbinsäure, Coenzym Q-10 und Glutathion eingesetzt. Dieses System eignet sich zur Simulation einer zellvermittelten, durch Lipoxygenase induzierten Oxidation von Lipiden wie sie z. B. infolge UV-Strahlung auftritt. Zur Bestimmung der antioxidativen Wirkung der einzelnen Substanzen wurde die im Zeitverlauf nach 8 bzw. 24 Stunden auftretende Menge an primären Abbauprodukten der Lipidperoxidation, den Lipidhydroperoxiden, mittels einer HPLC-Chemiluminiszenzmethode (E. Wieland et al., Fresenius J Anal Chem, 343, 62-63 (1992)) bestimmt. Trotz erheblich geringerer Konzentration von Idebenon im Ansatz (10 µmol/l) zeigte sich eine gegenüber Ascorbinsäure (50 µmol/l), alpha-Tocopherol (100µmol/l) und Glutathion (50 µmol/l) deutlich stärkere antioxidative Wirkung. Dies zeigte sich insbesondere bei Kombination der übrigen Antioxidantien mit Tocopherol. Nur Idebenon war hier in der Lage, den nach 24 Stunden auftretenden prooxidativen Effekt von Tocopherol-Oxidationsprodukten zuverlässig abzufangen (s. Tabelle 1). Insbesondere Ascorbinsäure, die bei bereits Tocopherol enthaltenden kosmetischen und topischen dermatologischen Zubereitungen gelegentlich hinzugefügt wird, zeigte in dieser Kombination im Zeitverlauf nur eine deutlich geringere antioxidative Wirkung. Die in den nachfolgenden Tabellen angegebenen Werte beziehen sich auf nmol gemessener Lipidhydroperoxide pro mg LDL-Protein.

**Tabelle 1**

| | ***Oxidationssystem (Bezugswerte)*** | | | |
|---|---|---|---|---|
| ***Zeit (Std.)*** | ***Mk ohne Zusatz*** | ***Mk* + *LDL*** | ***A* + *100 µmol*/*l α-Tocopherol*** | ***LDL ohne Zusatz*** |
| | | **(A)** | **(B)** | |
| 8 | 1,1 | 53,3 | 32,8 | 33,1 |
| 24 | 0 | 184,8 | 198,0 | 35,3 |

**Tabelle 2**

| | | ***Oxidationssystem (A) (*+ *1 Antioxidans)*** | | | | | |
|---|---|---|---|---|---|---|---|
| ***Zeit (Std.)*** | **A + *100 µmol*/*l α-Tocopherol*** | | ***A* + 10 *µmol*/*l Idebenon*** | ***A* + *50 µmol*/*l Ascorbinsäure*** | ***A* + *50 µmol*/*l Glutathion*** | | ***A* + *10 µmol*/*l Coenzym Q-10*** |
| 8 | 32,8 | | 0 | 0 | 51,7 | | 69 |
| 24 | 198,0 | | 0 | 1,7 | 119,0 | | 115,8 |

**Tabelle 3**

| | ***Oxidationssystem (B) (*+ *1 weiteres Antioxidans)*** | | | | | |
|---|---|---|---|---|---|---|
| ***Zeit (Std.)*** | | ***B* + *10 µmol*/*l Idebenon*** | ***B* + *50 µmol*/*l Ascorbinsäure*** | ***B* + *50 µmol*/*l Glutathion*** | ***B* + *10 µmol*/*l Coenzym Q-10*** | |
| 8 | | 1,9 | 1,7 | 23,3 | 26,8 | |
| 24 | | 2,6 | 25,3 | 129,8 | 143,7 | |

Auch andere Untersuchungen belegten, daß Idebenon und/oder seine Derivate einen Schutz gegen den Verlust von alpha-Tocopherol und Coenzym Q-10 im Gewebe unter prooxidativen Bedingungen darstellt (Schütz et al., Clin Biochem 30, 619 ff (1997)).

Weitere Bezeichnungen für die polymorphe Lichtdermatose sind PLD. PLE. Mallorca-Akne und eine Vielzahl von weiteren Bezeichnungen, wie sie in der Literatur (z. B. A. Voelekel et al., Zentralblatt Haut- und Geschlechtskrankheiten (1989),156, S. 2), angegeben sind.

Erythematöse Hauterscheinungen treten auch als Begleiterscheinungen bei gewissen Hauterkrankungen oder -Unregelmäßigkeiten auf. Beispielsweise ist der typische Hautausschlag beim Erscheinungsbild der Akne regelmäßig mehr oder weniger stark gerötet.

Hauptsächlich werden Antioxidantien als Schutzsubstanzen gegen den Verderb der sie enthaltenden Zubereitungen verwendet. Dennoch ist bekannt, daß auch in der menschlichen und tierischen Haut unerwünschte Oxidationsprozesse auftreten können. Solche Prozesse spielen eine wesentliche Rolle bei der Hautalterung.

Im Aufsatz "Skin Diseases Associated with Oxidative Injury" in "Oxidative Stress in Dermatology", S. 323 ff. (Marcel Decker Inc., New York, Basel, Hong Kong, Herausgeber: Jürgen Fuchs, Frankfurt, und Lester Packer, Berkeley/Californien), werden oxidative Schäden der Haut und ihre näheren Ursachen aufgeführt.

Antioxidantien sind Substanzen, welche Oxidationsprozesse verhindern bzw. welche die Autoxidation ungesättigte Verbindungen enthaltender Fette verhindern. Antioxidantien, welche auch auf dem Gebiete der Kosmetik und der Pharmazie Verwendung finden, sind beispielsweise alpha-Tocopherol insbesondere in Form des alpha-Tocopherylacetats, Sesamol, Gallensäurederivate, Burylhydroxyanisol und Butylhydroxytoluol.

Auch aus dem Grunde, solchen Reaktionen vorzubeugen, können kosmetischen Formulierungen zusätzlich Antioxidantien und/oder Radikalfänger einverleibt werden.

Zwar sind einige Antioxidantien und Radikalfänger bekannt. So ist bereits in den US-Patentschriften 4,144,325 und 4,248,861 sowie aus zahlreichen anderen Dokumenten vorgeschlagen worden, Vitamin E, eine Substanz mit bekannter antioxidativer Wirkung in Lichtschutzformulierungen einzusetzen, dennoch bleibt auch hier die erzielte Wirkung weit hinter der erhofften zurück.

Aufgabe der vorliegenden Erfindung war, die Nachteile des Standes der Technik zu beseitigen. Insbesondere sollten Wirkstoffe bzw. Zubereitungen, solche Wirkstoffe enthaltend, zur Verfügung gestellt werden, bei denen die Stabilität gegenüber Oxidation oder Fotooxidation verbessert ist.

Es war überraschend und für den Fachmann nicht vorherzusehen, daß die Verwendung von Idebenon und/oder seinen Derivaten als Antioxidans und/oder als Radikalfänger mit den zusätzlichen Funktionen als Stabilisator von Mitochondrienmembranen, Stimulator der Zellatmung und antiapoptotisches Agenz in kosmetischen oder topischen dermatologischen Zubereitungen den Nachteilen des Standes der Technik abhelfen kann.

Idebenon (6-(10-Hydroxydecyl)-2,3-dimethoxy-5-methyl-1,4-benzochinon) ist durch folgende Strukturformel gekennzeichnet:

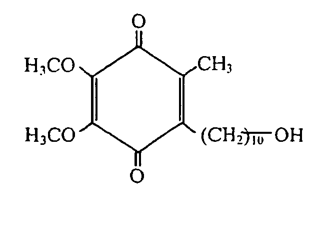

Beispiel eines hydrophilen Idebenonesters (eigene Synthese):

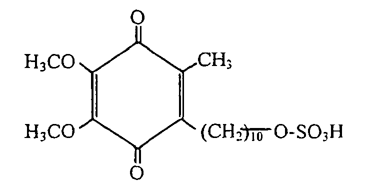

Zur Synthese des Idebenonesters (Idebenonsulfonsäure) wurde Idebenon mit PyridinSO₃ zur Reaktion gebracht und die Reaktion anschließend mit 1N Salzsäure gestoppt. Nach Ausschütteln der organischen Phase mit Äthylacetat wurde die organische Phase getrocknet und im Vakuum eingedampft. Der Rückstand wurde in Wasser gelöst und unlösliche Produkte abzentrifugiert. Der so gewonnene hydrophile Idebenonester eignet sich hervorragend zur erfindungsgemäßen Anwendung in wässrigen kosmetischen und dermatologischen Zubereitungen.

Die Schriften DE 3,049,039, EP 0,788,793 (13.08.1997), US 4,436,753, US 5,059,627, US 5,916,925 und WO 9,907,355(z.Bsp.) beschreiben zwar orale, parenterale oder perkutane Zubereitungen mit einem Gehalt an Idebenon oder seinen Derivaten zur Behandlung der Demenz, von Durchblutungsstörungen oder zur Induktion eines neuralen Wachstumsfaktors. Die Schrift JP 1,279,818 beschreibt die Verwendung von Idebenon und seinen Derivaten in verschiedenen Zubereitungen zur Färbung von Haaren. Idebenon zeigte bei oraler Verabreichung keine toxischen Effekte (Barkworth et al., Arzneim.-Forsch/Drug Res. 35 (II), 11, pp 1704 ff (1985)).

Kein Hinweis ist diesen Schriften aber zu entnehmen, welcher in die Richtung der vorliegenden Erfindung weisen könnte.

Es war für den Fachmann daher nicht vorauszusehen, daß das erfindungsgemäß verwendete Idenbenon und seine Derivate bzw. kosmetische oder dermatologische Zubereitungen, diese enthaltend
- besser als Antioxidans wirken
- besser als Radikalfänger wirken
als die Wirkstoffe und Zubereitungen des Standes der Technik. Ferner war nicht vorauszusehen gewesen, daß das Idebenon und seine Derivate in kosmetischen oder dermatologischen Zubereitungen höhere Stabilität aufweisen als vergleichbare Wirkstoffe, beispielsweise als Vitamin C oder Vitamin E.

Erfindungsgemäß ist daher die Verwendung von Idebenon und/oder seiner Derivate, wie z. B. der Idebenonsulfonsäure, oder natürlich auch die Verwendung anderer Derivate des Idebenons, als Antioxidans zur Stabilisierung von kosmetischen oder dermatologischen Zubereitungen gemäß Anspruch 1, insbesondere die als Zusatz entweder Vitamin A und/oder seine Derivate (z. B. all-E-Retinsäure, 9-Z-Retinsäure, 13-Z-Retinsäure, Retinal, Retinylester), Vitamin B und/oder seine Derivate, Vitamin C und/oder seine Derivate Vitamin E und/oder seine Derivate (z. B. Alpha-Tocopherolacetat und Hyaluronsaüre und/oder ihre Derivate einzeln oder in Kombination enthalten. Hierbei bezieht sich die stabilisierende Wirkung sowohl auf Geruch und Farbe als auch insbesondere auf den Wirkstoffgehalt der Zubereitung.

Die kosmetischen oder dermatologischen Formulierungen können wie üblich zusammengesetzt sein und zur Behandlung, der Pflege und der Reinigung der Haut und als Schminkprodukt in der dekorativen Kosmetik dienen. Sie enthalten bevorzugt 0,0001 Gew.-% bis 30 Gew.-%, bevorzugt 0,05 Gew.-% bis 5 Gew.-%, insbesondere 0,1-2,0 Gew.-%, bezogen auf das Gesamtgewicht des Mittels, an Idebenon und/oder seinen Derivaten.

Zur Anwendung werden die kosmetischen und dermatologischen Zubereitungen in der für Kosmetika üblichen Weise auf die Haut in ausreichender Menge aufgebracht.

Kosmetische und dermatologische Zubereitungen können in verschiedenen Formen vorliegen. So können sie z. B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsion, beispielsweise vom Typ Wasser-in-Öl-in-Wasser (W/O/W), ein Gel, einen festen Stift, eine Salbe oder auch ein Aerosol darstellen. Es ist auch vorteilhaft, Idebenon und/oder seine Derivate in verkapselter Form darzureichen, z. B. in Kollagenmatrices und anderen üblichen Verkapselungsmaterialien, z. B. als Celluloseverkapselungen, in Gelatine, Wachsmatrices oder liposomal verkapselt.

Es ist auch möglich und vorteilhaft im Sinne der vorliegenden Erfindung, ldebenon und/oder seine Derivate, hier insbesondere das Sulfat des Idebenons, in wäßrige Systeme bzw. Tensidzubereitungen zur Reinigung der Haut einzufügen.

Die kosmetischen und dermatologischen Zubereitungen können kosmetische Hilfsstoffe enthalten, wie sie üblicherweise in solchen Zubereitungen verwendet werden, z. B. Konservierungsmittel, Bakterizide, Parfüme, Substanzen zum Verhindern des Schäumens, Farbstoffe, Pigmente, die eine färbende Wirkung haben, Verdickungsmittel, oberflächenaktive Substanzen, Emulgatoren, weichmachende, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösemittel oder Silikonderivate.

Insbesondere können Idebenon und seine Derivate erfindungsgemäß auch mit anderen Antioxidantien und/oder Radikalfängern kombiniert werden.

Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidatitien gewählt aus der Gruppe bestehend aus Aminosäuren (z. B. Glycin, Histidin, Tyrosin, Tryptophan) und deren Derivate, Imidazole (z. B. Urocaninsäure) und deren Derivate, Peptide wie D,L-Carnosin, D-Carnosin, L-Carnosin und deren Derivate (z. B. Anserin), Carotinoide, Carotine (z. B. alpha-Carotin, beta-Carotin, Lycopin) und deren Derivate, Chlorogensäure und deren Derivate, Liponsäure und deren Derivate (z. B. Dihydroliponsäure), Aurothioglucose, Propylthiouracil und andere Thiole (z. B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, gamma-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z. B. Buthioninsulfoximine, Homocysteinsulfoximin, Buthioninsulfone, Penta-, Hexa-, Heptathioninsulfoximin) in sehr geringen, verträglichen Dosierungen (z. B. pmol bis µmol/kg), ferner (Metall)-Chelatoren (z. B. alpha-Hydroxyfettsäuren, Palmitinsäure, Phytinsäure, Lactoferrin), alpha-Hydroxysäuren (z. B. Citronensäure, Milchsäure, Apfelsäure, Mandelsäure), Huminsäure, Gallensäure, Gallenextrakte, Bilirubin, Biliverdin, EDTA, EGTA und deren Derivate, ungesättigte Fettsäuren und deren Derivate (z. B. gamma-Linolensäure, Linolsäure, Ölsäure), Folsäure und deren Derivate, Ubichinon und Ubichinol und deren Derivate, Vitamin C und Derivate (z. B. Ascorbylpalmitat, Mg-Ascorbylphosphat, Ascorbylacetat), Tocopherole und Derivate (z. B. Vitamin-E-acetat), Vitamin A und Derivate (z.B. Vitamin-A-palmitat) sowie Koniferylbenzoat des Benzoeharzes, Rutinsäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Sesamol, Sesamolin, Zink und dessen Derivate (z. B. ZnO, ZnSO₄), Selen und dessen Derivate (z. B. Selenmethionin), Stilbene und deren Derivate (z. B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den Zubereitungen beträgt vorzugsweise 0,0001 Gew.-% bis 30 Gew.-%, besonders bevorzugt 0,05 Gew.-% bis 20 Gew.-%, insbesondere 1 - 10 Gew.-%, bezogen auf das Gesamtgewicht der Zubereitung.

Sofern Vitamin E und/oder dessen Derivate das oder die zusätzlichen Antioxidantien darstellen, ist vorteilhaft, deren jeweilige Konzentration aus dem Bereich von 0,0001 - 20 Gew.-%, bezogen auf das Gesamtgewicht der Formulierung, zu wählen.

Sofern Vitamin A, bzw. Vitamin-A-Derivate, bzw. Carotine bzw. deren Derivate das oder die zusätzlichen Antioxidantien darstellen, ist vorteilhaft, deren jeweilige Konzentrationen aus dem Bereich von 0,0001 - 10 Gew.-%, bezogen auf das Gesamtgewicht der Formulierung, zu wählen.

Emulsionen sind vorteilhaft und enthalten z. B. die genannten Fette, Öle, Wachse und anderen Fettkörper, sowie Wasser und einen Emulgator, wie er üblicherweise für einen solchen Typ der Formulierung verwendet wird.

Die Lipidphase kann vorteilhaft gewählt werden aus folgender Substanzgruppe:
- Mineralöle, Mineralwachse;
- Öle, wie Triglyceride der Caprin- oder der Caprylsäure, ferner natürliche Öle wie z. B. Rizinusöl;
- Fette, Wachse und andere natürliche und synthetische Fettkörper, vorzugsweise Ester von Fettsäuren mit Alkoholen niedriger C-Zahl, z. B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat 2-Hexyldecylstearat, 2-Octyldodecylpalmitat, Oleyloleat, Oleylerucat, Erucyloleat, Erucylerucat sowie synthetische, halbsynthetische, und natürliche Gemische solcher Ester, z. B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12-18 C-Atomen. Die Fettsäuretriglyceride können beispielsweise vorteilhaft gewählt werden aus der Gruppe der synthetischen, halbsynthetischen und natürlichen Öle, z. B. Olivenlöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid, Dicaprylylether.

Besonders vorteilhaft sind Mischungen aus C₁₂₋₁₅-Alkylbenzoat und 2-Ethylhexylisostearat, Mischungen aus C₁₂₋₁₅-Alkylbenzoat und Isotridecylisononanoat sowie Mischungen aus C₁₂₋₁₅-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Oktamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimediylsiloxan, Poly(methylphenylsiloxan).

Besonders vorteilhaft sind ferner Mischungen aus Cyclomethicon und Isotridecylisononanoat, aus Cyclomethicon und 2-Ethylheaylisostearat.

Die wäßrige Phase der Zubereitungen enthält gegebenenfalls vorteilhaft Alkohole, Diole oder Polyole niedriger C-Zahl, sowie deren Ether, vorzugsweise Ethanol, Isopropanol Propylenglykol, Glycerin, Ethylenglykol, Ethylenglykolmonoethyl- oder -monobutylether, Propylenglykolmonomethyl, monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, jeweils einzeln oder in Kombination.

Insbesondere werden Gemische der vorstehend genannten Lösungsmittel verwendet. Bei alkoholischen Lösemitteln kann Wasser ein weiterer Bestandteil sein.

Emulsionen sind vorteilhaft und enthalten z. B. die genannten Fette, Öle, Wachse und anderen Fettkörper, sowie Wasser und einen Emulgator, wie er üblicherweise für einen solchen Typ der Formulierung verwendet wird.

Gele enthalten üblicherweise Alkohole niedriger C-Zahl, z. B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugsweise ein Polyacrylat ist.

Als Treibmittel für aus Aerosolbehältern versprühbare Zubereitungen sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Vorteilhaft können die Zubereitungen außerdem Substanzen enthalten, die UV-Strahlung im UVB-Bereich absorbieren, wobei die Gesamtmenge der Filtersubstanzen z. B. 0,1 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die die Haut vor dem gesamten Bereich der ultravioletten Strahlung schützen. Sie können auch als Sonnenschutzmittel für die Haut dienen.

Enthalten die Zubereitungen UVB-Filtersubstanzen, können diese öllöslich oder wasserlöslich sein. Vorteilhafte öllösliche UVB-Filter sind z. B.:
- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomentylester,
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester, - 2,4,6-Trianilino(p-carbo-2'-ethyl- 1'-hexyloxy)-1,3,5-triazin.

Vorteilhafte wasserlösliche UVB-Filter sind z. B.:
- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z. B. 4-(2-Oxo-3-bornyliden-rnethyl)benzolsulfon-säure, 2-Methyl-5-(2-oxo-3-bornylidenmethyl)sulfonsäure und ihre Salze sowie das 1,4-di(2-oxo-10-Sulfo-3-bornylidenmethyl)-Benzol und dessen Salze (die entsprechenden 10-Sulfatoverbindungen, beispielsweise das entsprechende Natrium-, Kalium- oder Triethanolammonium-Salz), auch als Benzol-1,4-di(2-oxo-1-bornylidenmethyl)-10-Sulfonsäure bezeichnet.

Die Liste der genannten UVB-Filter, die in Kombination mit den Wirkstoffkombinationen verwendet werden können, soll selbstverständlich nicht limitierend sein.

Gegenstand der Erfindung ist auch die Verwendung einer Kombination des Idebenons und/oder seiner Derivate mit mindestens einem UVB-Filter als Antioxidans bzw. die Verwendung einer Kombination des Idebenons und/oder seiner Derivate mit mindestens einem UVB-Filter als Antioxidans in einer kosmetischen oder dermatologischen Zubereitung.

Es kann auch von Vorteil sein, Idebenon und/oder seine Derivate mit UVA-Filtern zu kombinieren, die bisher üblicherweise in kosmetischen Zubereitungen enthalten sind. Bei diesen Substanzen handelt es sich vorzugsweise um Derivate des Dibenzoylmethans, insbesondere um 1-(4'-tert.Butylphenyl)-3-(4' methoxyphenyl)propan-1,3-dion und um 1-Phenyl-3-(4'-isopropylphenyl)propan-1,3-dion. Auch diese Kombinationen bzw. Zubereitungen, die diese Kombinationen enthalten, sind Gegenstand der Erfindung. Es können die für die UVB-Kombination verwendeten Mengen eingesetzt werden.

Gegenstand der Erfindung ist auch die Verwendung einer Kombination von Idebenon und/oder seiner Derivate mit mindestens einem UVA-Filter als Antioxidans bzw. die Verwendung einer Kombination der erfindungsgemäßen Wirkstoffkombinationen mit mindestens einem UVA-Filter als Antioxidans in einer kosmetischen oder dermatologischen Zubereitung.

Gegenstand der Erfindung ist auch die Verwendung einer Kombination aus Idebenon und/oder seiner Derivate mit mindestens einem UVA-Filter und mindestens einem UVB-Filter als Antioxidans bzw. die Verwendung einer Kombination aus Idebenon und/oder seiner Derivate mit mindestens einem UVA-Filter und mindestens einem UVB-Filter als Antioxidans in einer kosmetischen oder dermatologischen Zubereitung.

Kosmetische und dermatologische Zubereitungen mit einem wirksamen Gehalt an Idebenon und/oder seinen Derivaten können auch anorganische Pigmente enthalten, die üblicherweise in der Kosmetik zum Schutz der Haut vor UV-Strahlen verwendet werden. Dabei handelt es sich um Oxide des Titans, Zinks, Zirkoniums, Siliciums, Mangans, Cers und Mischungen davon, sowie Abwandlungen, bei denen die Oxide die aktiven Agentien sind. Besonders bevorzugt handelt es sich um Pigmente auf der Basis von Titandioxid.

Auch diese Kombinationen von UVA-Filter und Pigment bzw. Zubereitungen, die diese Kombination enthalten, sind Gegenstand der Erfindung. Es können die für die vorstehenden Kombinationen genannten Mengen verwendet werden.

Kosmetische Zubereitungen, die ein Hautreinigungsmittel oder Shampoonierungsmittel darstellen, enthalten vorzugsweise mindestens eine anionische, nicht-ionische oder amphotere oberflächenaktive Substanz, oder auch Gemische aus solchen Substanzen, Idebenon und/oder seine Derivate im wäßrigen Medium und Hilfsmittel, wie sie üblicherweise dafür verwendet werden. Die oberflächenaktive Substanz bzw. die Gemische aus diesen Substanzen können in einer Konzentration zwischen 1 Gew.-% und 50 Gew.-% in dem Shampoonierungsmittel vorliegen.

Diese kosmetischen oder dermatologischen Zubereitungen können auch Aerosole mit den üblicherweise dafür verwendeten Hilfsmitteln darstellen.

Wäßrige kosmetische Reinigungsmittel oder für die wäßrige Reinigung bestimmte wasserarme oder wasserfreie Reinigungsmittelkonzentrate können anionische, nichtionische und/oder amphotere Tenside enthalten, beispielsweise
- herkömmliche Seifen, z. B. Fettsäuresalze des Natriums
- Alkylsulfate, Alkylethersulfate, Alkan- und Alkylbenzolsulfonate
- Sulfoacetate
- Sulfobetaine
- Sarcosinate
- Amidosulfobetaine
- Sulfosuccinate
- Sulfobernsteinsäurehalbester
- Alkylethercarboxylate
- Eiweiß-Fettsäure-Kondensate
- Alkylbetaine und Amidobetaine
- Fettsäurealkanolamide
- Polyglycolether-Derivate.

Kosmetische Zubereitungen, die kosmetische Reinigungszubereitungen für die Haut darstellen, können in flüssiger oder fester Form vorliegen. Sie enthalten neben Idebenon und/oder seinen Derivaten vorzugsweise mindestens eine anionische, nicht-ionische oder amphotere oberflächenaktive Substanz oder Gemische daraus, gswünschtenfalls einen oder mehrere Elektrolyten und Hilfsmittel, wie sie üblicherweise dafür verwendet werden. Die oberflächenaktive Substanz kann in einer Konzentration zwischen 0.001 und 99,999 Gew.-% in den Reinigungszubereitungen vorliegen, bezogen auf das Gesamtgewicht der Zubereitungen.

Kosmetische Zubereitungen die ein Shampoonierungsmittel darstellen, enthalten neben einem wirksamen Gehalt an Idebenon und/oder seinen Derivaten vorzugsweise eine anionische, nicht-anionische oder amphotere oberflächenaktive Substanz oder Gemische daraus, gegebenenfalls einen erfindungsgemäßen Elektrolyten und Hilfsmittel, wie sie üblicherweise dafür verwendet werden. Die oberflächenaktive Substanz kann in einer Konzentration zwischen 0,001 Gew.-% und 99,999 Gew.-% in dem Shampoonierungsmittel vorliegen.

Die Zusammensetzungen enthalten außer den vorgenannten Tensiden Wasser und gegebenenfalls die in der Kosmetik üblichen Zusatzstoffe, beispielsweise Parfüm, Verdicker, Farbstoffe, Desodorantien, antimikrobielle Stoffe, rückfettende Agentica, Komplexierungs- und Sequestrierungsagentien, Perlglanzagentien, Pflanzenextrakte, Vitamine und oder deren Derivate, Wirkstoffe und dergleichen.

Die vorliegende Erfindung umfaßt ein Verfahren zum Schutze kosmetischer oder dermatologischer Zubereitungen gegen Oxidation oder Photooxidation, wobei diese Zubereitungen z. B. Zubereitungen zur Behandlung und Pflege der Haare darstellen, insbesondere Haarlacke, Shampoonierungsmittel, ferner Schminkprodukte wie z. B. Nagellacke, Lippenstifte, Teintgrundlagen, Wasch- und Duschzubereitungen, Cremes zur Behandlung oder Pflege der Haut oder um sämtliche anderen kosmetischen Zubereitungen handelt, deren Bestandteile Stabilitätsprobleme aufgrund von Oxidation bzw Photooxidation bei der Lagerung mit sich bringen können, dadurch gekennzeichnet, daß die kosmetischen Zubereitungen einen wirksamen Gehalt an Idebenon und/oder seinen Derivaten aufweisen.

Vorzugsweise beträgt die Menge an Idebenon und/oder seinen Derivaten in diesen Zubereitungen 0,0001 - 30 Gew.-%, bevorzugt 0,05 - 5 Gew.-%, insbesondere 0,1 - 2,0 Gew.-%, bezogen auf das Gesamtgewicht der Zubereitungen.

Die nachfolgenden Beispiele sollen die vorliegende Erfindung verdeutlichen, ohne sie einzuschränken. Alle Mengenangaben, Anteile und Prozentangaben sind, soweit nicht anders angegeben, auf das Gewicht und die Gesamtmenge bzw. auf das Gesamtgewicht der Zubereitungen bezogen.

### Beispiel 1

| O/W Lotion | |
|---|---|
| | Gew.-% |
| Paraffinöl (DAB 9) | 8,00 |
| Petrolatum | 4,00 |
| Ocrylmethoaycinnamat | 5,00 |
| Isopropylpalmitat | 3,00 |
| Glycerin | 3,00 |
| Cetylstearylalkohol | 2,00 |
| Butylmethoxydibenzoylmethan | 1,00 |
| PEG-40 Rizinusöl | 0,50 |
| Natriumcetystearylsulfat | 0.50 |
| Idebenon | 0,50 |
| Hyaluronat | 0,50 |
| Natrium Carbomer | 0,40 |
| Alpha-Tocopherol | 0,20 |
| Konservierungsmittel, Farbstoffe, Parfüm | q.s. |
| Wasser | ad 100,00 |

### Beispiel 2

| O/W Creme | |
|---|---|
| | Gew.-% |
| Paraffinöl (DAB 9) | 7,00 |
| Avocadoöl | 4,00 |
| Natriumlactat | 3,00 |
| Glycerin | 3,00 |
| Glycerylmonostearat | 2,00 |
| Titandioxid | 1,00 |
| Idebenon | 0,50 |
| Hyaluronat | 0,50 |
| Konservierungsmittel, Farbstoffe, Parfüm | q.s. |
| Wasser | ad 100,00 |

### Beispiel 3

| W/O Creme | |
|---|---|
| | Gew.-% |
| Paraffinöl (DAB 9) | 10,00 |
| Caprylsäure-/Caprinsäure Triglycerid | 5,00 |
| Buxus Chinensis | 5,00 |
| Avocadoöl | 4,00 |
| PEG-40 hydriertes Rizinusöl | 4,00 |
| 1,2 Propylenglykol | 3,00 |
| Vaseline | 3,00 |
| Idebenonsulfonat | 1,00 |
| Alpha-Tocopherolacetat | 1,00 |
| Retinol 10 CM | 1,00 |
| Parabene | 0,50 |
| Idebenon | 0,50 |
| Hyaluronat | 0,50 |
| Alpha-Tocopherol | 0,50 |
| Konservierungsmittel, Farbstoffe, Parfüm | q.s. |
| Wasser | ad 100,00 |

### Beispiel 4

| Lippenpflegestift | |
|---|---|
| | Gew.-% |
| Petrolatum | 40,00 |
| Ceresin | 8,00 |
| Hydriertes Rizinusöl | 4,00 |
| Bienenwachs | 4,00 |
| Carnaubawachs | 2,00 |
| Idebenon | 0,50 |
| β-Carotin | 0,10 |
| Konservierungsmittel, Farbstoffe, Parfüm | q.s. |
| Paraffinöl | ad 100,00 |

### Beispiel 5

| Lippenpflegestift | |
|---|---|
| | Gew.-% |
| Petrolatum | 40,00 |
| Isopropyllanolat | 10,00 |
| Bienenwachs, gebleicht | 9,00 |
| Acetyliertes Lanolin | 4,00 |
| Carnaubawachs | 4,00 |
| Glycerin | 3,00 |
| Idebenon | 0,50 |
| Alpha-Tocopherolacetat | 0,10 |
| Konservierungsmittel, Farbstoffe, Parfüm | q.s. |
| Paraffinöl | ad 100,00 |

### Beispiel 6

| Haarwasser | |
|---|---|
| | Gew.-% |
| Ethanol | 40,00 |
| Idebenon | 0,50 |
| Alpha-Tocopherolacetat | 0,50 |
| PEG-40 hydriertes Rizinusöl | 0,20 |
| Disopropyladipat | 0,10 |
| Konservierungsmittel, Farbstoffe, Parfüm | q.s. |
| Wasser | ad 100,00 |

### Beispiel 7

| Liposomenhaltiges Gel | |
|---|---|
| | Gew-% |
| Lecithin | 6,00 |
| Sorbitol | 3,00 |
| Hydrolysiertes Kollagen | 2,00 |
| Xanthan Gummi | 1,40 |
| Natriumcitrat | 0,50 |
| Natrium PCA | 0,50 |
| Idebenon | 0,50 |
| Glycin | 0,20 |
| Harnstoff | 0,20 |
| Alpha-Tocopherol | 0,20 |
| Biotin | 0,08 |
| Konservierungsmittel, Farbstoffe, Parfüm | q.s. |
| Wasser | ad 100,00 |

### Beispiel 8

| Sonnenschutzemulsion | |
|---|---|
| | Gew.-% |
| Octylmethoxycinnamat | 5,00 |
| Rizinusöl | 4,00 |
| Glycerin | 3,00 |
| Octylstearat | 3,00 |
| Laurylmethicon Copolyol | 2,00 |
| Cyclomethicone | 2,00 |
| Cetylstearylalkohol | 1,80 |
| Na₃HEDTA | 1,50 |
| Glycerollanolat | 1,00 |
| Burylmethoxydibenzoylmethan | 1,00 |
| Idebenon | 0,50 |
| PEG-40 hydriertes Rizinusöl | 0,30 |
| Natrium Cerylstearylsulfat | 0,30 |
| Acrylamid/Natriumacrylat Copolymer | 0,30 |
| Alpha-Tocopherol | 0,20 |
| Caprylsäure-/Caprinsäure Triglycerid | 0,10 |
| Konservierungsmittel, Farbstoffe, Parfüm | q.s. |
| Wasser | ad 100,00 |

### Beispiel 9

| Sonnenschutzemulsion | |
|---|---|
| | Gew.-% |
| Caprylsäure-/Caprinsäure Triglycerid | 6,00 |
| Octytmethoxycinnamat | 5,00 |
| Butylmethoxydibenzoylmethan | 4,00 |
| PEG 22-Dodecyl Copolymer | 3,00 |
| Paraffinöl (DAB9) | 2,00 |
| Cyclomethicone | 2,00 |
| Idebenon | 0,50 |
| Alpha-Tocopherolacetat | 0,50 |
| Na₃HEDTA | 0,50 |
| Cetyldimethicon Copolyol | 0,20 |
| Konservierungsmittel, Farbstoffe, Parfüm | q.s. |
| Wasser | ad 100,00 |

### Beispiel 10

| Sonnenschutzemulsion | |
|---|---|
| | Gew.-% |
| Octylmethoxycinnamat | 4,00 |
| Rizinusöl | 4,00 |
| Octylstearat | 3,00 |
| Glycerin | 3,00 |
| Cyclomethicone | 2,00 |
| Laurylmethicon Copolyol | 2,00 |
| Cetylstearylalkohol | 1,70 |
| Na₃HEDTA | 1,50 |
| Glycerollanolat | 1,00 |
| Burylmethoaydibenzoylmethan | 1,00 |
| Alpha-Tocopherolacetat | 1,00 |
| Idebenon | 0,50 |
| PEG-40 hydriertes Rizinusöl | 0,40 |
| Natrium Cetylstearylsulfat | 0,30 |
| Acrylamid/Natriumacrylat Copolymer | 0,30 |
| Hydroxypropylmethylcellulose | 0,30 |
| Caprylsäure-/Caprinsäure Triglycerid | 0,10 |
| Konservierungsmittel, Farbstoffe, Parfüm | q.s. |
| Wasser | ad 100,00 |

### Beispiel 11

| Gel | |
|---|---|
| | Gew.-% |
| Triethanolamin | 3,00 |
| Carbopol 934 P | 2,00 |
| Hydrolisiertes Kollagen | 2,00 |
| Glycerin | 2,00 |
| Natrium PCA | 0,50 |
| Idebenon | 0,50 |
| Alpha-Tocopherolacetat | 0,20 |
| Konservierunssmittel, Farbstoffe, Parfüm | q.s. |
| Wasser | ad 100,00 |

### Beispiel 12

| Sprayformulierung | |
|---|---|
| | Gew.-% |
| Ethanol | 30,00 |
| Idebenon | 0,50 |
| Alpha-Tocopherolacetat | 0,10 |
| Konservierungsmittel, Farbstoffe, Parfüm | q.s. |
| Propan/Butan 25/75 | ad 100,00 |

## Patentansprüche

1. Verwendung von Idebenon und/oder seinen Derivaten zum Schutz von kosmetischen und dermatologischen Zubereitungen und deren Bestandteilen vor Oxidation oder Fotooxidation.

2. Verwendung nach Anspruch 1, wobei das Derivat des Idebenons als hydrophiler Ester vorliegt.

3. Verwendung nach mindestens einem der vorangehenden Ansprüche, wobei das Derivat des Idebenons als Sulfonsäureester vorliegt.

4. Verwendung nach mindestens einem der vorangehenden Ansprüche, wobei das Derivat des Idebenons als Ester des Idebenons und/oder seiner Derivate mit Glykosaminglykanen und/oder ihren Derivaten oder mit Hyaluronidase-Inhibitoren vorliegt.

5. Verwendung nach mindestens einem der vorangehenden Ansprüche, wobei Idebenon und/oder seine Derivate in Kombination mit anderen Antioxidantien enthalten ist.

6. Verwendung nach mindestens einem der vorangehenden Ansprüche, wobei Idebenon und/oder seine Derivate in Konzentrationen von 0,0001 Gew.-% bis 30 Gew.-%, bevorzugt von 0,05 Gew.-% bis 5 Gew.-%, insbesondere von 0,1 Gew.-% bis 2,0 Gew.-%, bezogen auf das Gesamtgewicht der Zubereitung, vorliegt.

7. Verwendung nach mindestens einem der vorangehenden Ansprüche, wobei die zu schützenden Bestandteile Vitamin A und/oder seine Derivate, Vitamin B und/oder seine Derivate, Vitamin C und/oder seine Derivate, Vitamin E und/oder seine Derivate, und Hyaluronsäure und/oder ihre Derivate, einzeln oder in Kombination, sind.

## Claims

1. Use of idebenone and/or its derivatives for protection of cosmetic and dermatological preparations and their components against oxidation and photo oxidation.

2. Use according to claim 1, wherein the derivative of the idebenone is present as hydrophilic ester.

3. Use according to any one of the preceeding claims, wherein the derivative of the idebenone is present as sulphonic acid ester.

4. Use according to any one of the preceeding claims, wherein the derivative of idebenone is present as an ester of idebenone and/or its derivatives with glycosamionoglycans and/or their derivatives or with hyaluronidase inhibitors.

5. Use according to any one of the preceeding claims, wherein the idebenone and/or its derivatives is contained in combination with other antioxidants.

6. Use according to any one of the preceeding claims, wherein the idebenone and/or its derivatives is present in concentrations of 0.0001% by weight to 30% by weight, preferably from 0.05% by weight to 5% by weight, in particular from 0.1% by weight to 2.0% by weight, based on the total weight weight of the preparation.

7. Use according to any one of the preceeding claims, wherein the components to be protected are vitamin A and/or its derivatives, vitamin B and/or its derivatives, vitamin C and/or its derivatives, vitamin E and/or its derivatives and hyaluronic acid and/or its derivatives, separately or in combination.

## Revendications

1. Utilisation d'idébénone et/ou de ses dérivés pour la protection de préparations cosmétiques et dermatologiques et de leurs constituants contre l'oxydation ou la photo-oxydation.

2. Utilisation selon la revendication 1, dans laquelle le dérivé d'idébénone est présent sous forme d'ester hydrophile.

3. Utilisation selon l'une au moins des revendications précédentes, dans laquelle le dérivé d'idébénone est présent sous forme d'ester d'acide sulfonique.

4. Utilisation selon l'une au moins des revendications précédentes, dans laquelle le dérivé d'idébénone est présent sous forme d'ester de l'idébénone et/ou de ses dérivés avec des glycosaminoglycanes et/ou leurs dérivés ou bien avec des inhibiteurs de la hyaluronidase.

5. Utilisation selon l'une au moins des revendications précédentes, dans laquelle l'idébénone et/ou ses dérivés sont contenus en combinaison avec d'autres antioxydants.

6. Utilisation selon l'une au moins des revendications précédentes, dans laquelle l'idébénone et/ou ses dérivés sont présents à des concentrations allant de 0,0001 % en poids à 30% en poids, de préférence de 0,05% en poids à 5% en poids et en particulier de 0,1% en poids à 2,0% en poids, ramenées au poids total de la préparation.

7. Utilisation selon l'une au moins des revendications précédentes, dans laquelle les constituants à protéger sont la vitamine A et/ou ses dérivés, la vitamine B et/ou ses dérivés, la vitamine C et/ou ses dérivés, la vitamine E et/ou ses dérivés et l'acide hyaluronique et/ou ses dérivés, seuls ou en combinaison.
